# EUROPEAN PATENT APPLICATION

(11) **EP 4 074 698 A1**
(43) Date of publication of application: **19.10.2022**
(21) Application number: 21169034.2
(22) Date of filing: 17.04.2021
(51) Int. Cl.: C07D 339/08

(54) **A TRIFLUOROMETHYL THIANTHRENIUM COMPOUND, PROCESS FOR PREPARING THE SAME AND THE USE THEREOF**

(71) Applicant: Studiengesellschaft Kohle mbH, 45470 Mülheim (DE)
(72) Inventor: RITTER, Tobias, 45470 Muelheim (DE); Häring, Andreas, 84051 Mirskofen (DE)

(57) **Abstract**

The present inventions refers a novel trifluoromethyl thianthrenium compound referred to as TT-CF₃⁺X⁻, a process for preparing the same and the use thereof for trifluoromethylating organic compounds.

## Description

The present inventions refers a novel trifluoromethyl thianthrenium compound, in the following referred to as TT-CF₃⁺X⁻, a process for preparing the same and the use thereof for trifluoromethylating organic compounds.

More than 65 pharmaceuticals containing the trifluoromethyl substituent have been approved by the FDA so far. Because no known sources exist in nature, the trifluoromethyl group must be installed into designed molecules by humans. Depending on the desired chemical transformation, different reagents for electrophilic, radical, or nucleophilic trifluoromethylation are employed.

Synthesis of simple trifluoromethyl-substituted molecules often employ harsh reagents, such as HF, F₂, or SF₄, that typically install the fluorine atoms on a (functionalized) methyl group already present in the molecule. For the functionalization of more complex small molecules, the use of CF₃ reagents of appropriate reactivity is more common, and, depending on the reaction, can be accomplished by nucleophilic, electrophilic, or radical CF₃ sources. The CF₃ radial can be produced from metastable reagents, such as trifluoroiodomethane, zinc triflinate, and sodium triflinate, via single electron chemistry. Nucleophilic trifluoromethylating reagents are formal precursors of the unstable CF₃ anion.

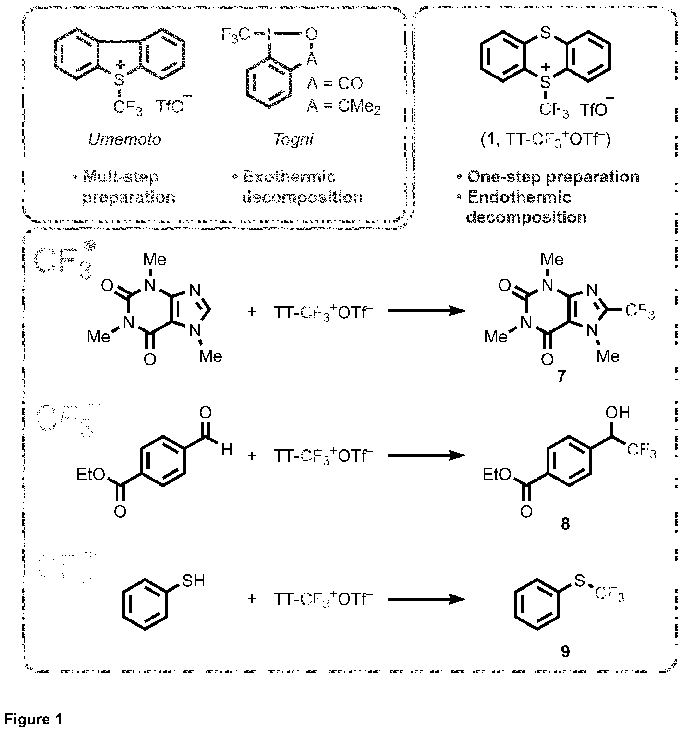

Common CF₃ anion precursors are PhSO₂CF₃ and TMS-CF₃, which form nucleophilic anionic complexes when activated with alkoxide or fluoride. Electrophilic trifluoromethylating reagents can often be used as both CF₃⁺ and CF₃ radical sources. Most electrophilic trifluoromethylating reagents are based on hypervalent iodine or chalcogenide reagents. The *Togni* reagents 1/11 and *Umemoto's* reagent (as shown in Figure 1) are the most prominent representatives of these classes, now commercially available, and of large synthetic value.

Given the broad interest in trifluoromethylation chemistry, the inventors introduce here the new reagent trifluoromethyl thianthrenium triflate (1, TT-CF₃⁺OTf⁻) or in combination with another anion. In contrast to other sulfonium-based trifluoromethylating reagents such as the *Umemoto's* reagent, the inventive TT-CF₃⁺OTf⁻ is readily accessible in a single step from inexpensive starting materials. It shows undiminished reactivity after storage for one year in ambient atmosphere. The new trifluoromethylating reagent engages in electrophilic, radical, and nucleophilic trifluoromethylation.

Because of its simple preparation, handling, high reactivity, and broad tolerance of functional groups present in complex molecules, as well as its divergent reactivity, the inventors expect that TT-CF₃⁺X⁻, in particular TT-CF₃⁺OTf⁻ will find widespread utility in future reaction chemistry development. The main difference of the inventive TT-CF₃⁺X⁻ when compared to most other sulfonium-based reagents is its simple one-step synthesis protocol; in contrast, the practical synthesis for the classical *Umemoto's* reagent requires nine steps. The fundamental difference to the *Togni* reagents is the higher reduction potential of the inventive TT-CF₃⁺X⁻, a consequence of the positive charge that can result in complementary reactivity in single electron transfer reactions when compared to the λ³-iodane compounds.

Thus the present invention is directed to an optionally substituted trifluoromethyl thrianthrenium derivative TT-CF₃⁺X⁻.In more detail, the present invention is thus directed, in a first aspect, to a thianthrene derivative of the Formula (I): 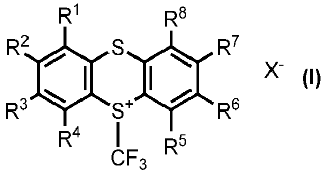 wherein R¹ to R⁸ may be the same or different and are each selected from hydrogen, halogen, or a C₁ to C₆ alkyl group, which may be substituted by at least one halogen, and wherein X⁻ is an anion, selected from F⁻, Cl⁻, triflate⁻ , BF₄⁻, SbF₆⁻, PF₆⁻, ClO₄⁻, 0.5 SO₄²⁻, or NO₃⁻.

In an embodiment of the thianthrene derivative of the Formula (I), R¹ to R⁸ may be the same or different and are each selected from hydrogen, Cl or F and X⁻ is an anion as defined before, preferably triflate, or BF₄.

In another embodiment of the thianthrene derivative of the Formula (I), R¹ to R⁸ are hydrogen, and X⁻ is an anion as defined before, preferably a triflate or BF₄ anion.

In the above formulae, X⁻ represents an anion selected from F⁻, Cl⁻, triflate⁻ , BF₄⁻, SbF₆⁻, PF₆⁻, ClO₄⁻, 0.5 SO₄²⁻, or NO₃⁻, and similar anions which result in a stable ion pair with the trifluoromethyl thrianthrenium cation.

In a second aspect, the present invention is directed to a process for preparing said trifluoromethyl thianthrenium compound of the Formula (I), whereby an optionally substituted thianthrene of the Formula (II) is reacted with a trifluoromethyl group transfer agent such as triflic acid anhydride in an organic solvent whereby a thianthrenium compound of the Formula (I) is obtained: 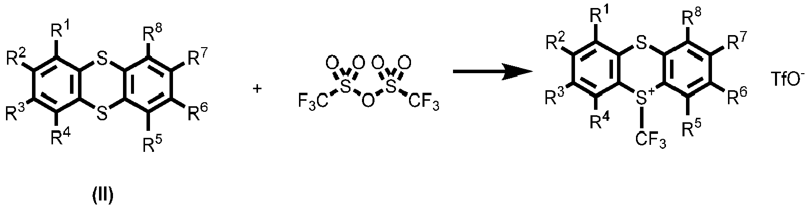 wherein R¹ to R⁸ may be the same or different and are each selected from hydrogen, halogen, or a C₁ to C₆ alkyl group, which may be substituted by at least one halogen, preferably F or Cl.

The triflate anion can be easily exchanged by another anion, selected from F⁻, Cl⁻, BF₄⁻, SbF₆⁻, PF₆⁻, ClO₄⁻, 0.5 SO₄²⁻, NO₃⁻, for example in contact with an aqueous solution of the other anion so that other trifluoromethyl thianthrenium anion pairs can be obtained, and wherein X⁻ is an anion, selected from F⁻, Cl⁻, triflate⁻ , BF₄⁻, SbF₆⁻,. PF₆⁻, ClO₄⁻, 0.5 SO₄²⁻, or NO₃⁻.

The inventive TT-CF₃⁺X⁻ is useful for trifluoromethylating organic hydrocarbons. Thus, the present invention is furthermore directed, in a third aspect, to the use of a trifluoromethyl thianthrenium compound of the Formula (I) as a transfer agent for transferring a trifluoromethyl group to a hydrocarbon compound, in particular to an aliphatic hydrocarbon, an aromatic hydrocarbon or a heteroaromatic hydrocarbon.

The inventive TT-CF₃⁺X⁻ is furthermore useful for transferring a trifluoromethyl group to a hydrocarbon compound, wherein said hydrocarbon compound has an at least one alkenyl moiety or C=O moiety and/or is substituted by at least one group selected from -B(OR)₂; -SR or -OR wherein R is hydrogen or C₁ to C₃ alkyl. Depending on the compound to be trifluoromethylated, the reaction can proceed via an electrophilic, radical or nucleophilic pathway.

In the inventive process for preparing the inventive TT-CF₃⁺X⁻ or the use thereof for trifluoromethylation, the choice of the organic solvent is not critical as long as it is an aprotic organic solvent selected from acetonitrile, other nitriles, chlorinated hydrocarbons, or other aprotic solvents, or mixtures thereof. The reaction conditions are also not critical and the reaction is usually carried out at a temperature between -78°C and 50°C, preferably 0°C to 30°C, under ambient pressure and optionally under an inert gas atmosphere.

In the inventive process for transferring the trifluoromethyl group, the aromatic hydrocarbon or heteroaromatic hydrocarbon may be a monocyclic or polycyclic, aromatic or heteroaromatic hydrocarbon having 5 to 22 carbon atoms, which may be unsubstituted or substituted by one of more substituents selected from saturated or unsaturated, straight chain or branched aliphatic hydrocarbons having 1 to 20 carbon atoms, aromatic or heteroaromatic hydrocarbons having 5 to 22 carbon atoms, heterosubstituents, or which may be part of a cyclic hydrocarbon ring system (carbocyclic) with 5 to 30 carbon atoms and/or heteroatoms. The definition for said aliphatic hydrocarbons may include one or more heteroatoms such O, N, S in the hydrocarbon chain.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "aliphatic" includes both saturated and unsaturated, straight chain (*i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain, branched or cyclic saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (e.g., -CH₃). In certain embodiments, the alkyl group is a substituted C₁₋₁₀ alkyl.

"Aryl" refers to a radical of a monocyclic or polycyclic (*e.g.*, bicyclic or tricyclic) 4n+2 aromatic ring system (*e.g.*, having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.,* naphthyl such as 1-naphthyl and 2-naphthyl). In some embodiments, an aryl group has fourteen ring carbon atoms ("C₁₄ aryl"; *e.g.,* anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" refers to a radical of a 5-14 membered monocyclic or bicyclic 4n+2 aromatic ring system (*e.g.*, having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g.*, indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g.*, 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

"Unsaturated" or "partially unsaturated" refers to a group that includes at least one double or triple bond. A "partially unsaturated" ring system is further intended to encompass rings having multiple sites of unsaturation, but is not intended to include aromatic groups (*e.g.*, aryl or heteroaryl groups) as herein defined. Likewise, "saturated" refers to a group that does not contain a double or triple bond, *i.e.,* contains all single bonds.

Alkyl, alkenyl, alkynyl, carbocyclyl, heterocyclyl, aryl, and heteroaryl groups, which are divalent bridging groups, are further referred to using the suffix -ene, *e.g.,* alkylene, alkenylene, alkynylene, carbocyclylene, heterocyclylene, arylene, and heteroarylene.

An atom, moiety, or group described herein may be unsubstituted or substituted, as valency permits, unless otherwise provided expressly. The term "optionally substituted" refers to substituted or unsubstituted.

Alkyl, alkenyl, alkynyl, carbocyclyl, heterocyclyl, aryl, and heteroaryl groups are optionally substituted (*e.g.*, "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. For purposes of this invention, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

Exemplary substituents include, but are not limited to, halogen, -CN, -NO₂, -N₃, -SO₂H,-SO₃H, -OH, -O-alkyl, -N-dialkyl, -SH, -S.alkyl, -C(=O)alkyl,-CO₂H, -CHO.

"Halo" or "halogen" refers to fluorine (fluoro, -F), chlorine (chloro, -CI), bromine (bromo, - Br), or iodine (iodo, -I).

"Acyl" refers to a moiety selected from the group consisting of -C(=O)R^{aa},-CHO, -CO₂R^{aa}, -C(=O)N(R^{bb})₂, -C(=NR^{bb})R^{aa}, -C(=NR^{bb})OR^{aa}, -C(=NR^{bb})N(R^{bb})₂, -C(=O)NR^{bb}SO₂R^{aa},-C(=S)6N(R^{bb})₂, -C(=O)SR^{aa}, or -C(=S)SR^{aa}, wherein R^{aa} and R^{bb} are as defined herein.

The term "catalysis," "catalyze," or "catalytic" refers to the increase in rate of a reaction due to the participation of a substance called a "catalyst." In certain embodiments, the amount and nature of a catalyst remains essentially unchanged during a reaction. In certain embodiments, a catalyst is regenerated, or the nature of a catalyst is essentially restored after a reaction. A catalyst may participate in multiple chemical transformations. The effect of a catalyst may vary due to the presence of other substances known as inhibitors or poisons (which reduce the catalytic activity) or promoters (which increase the activity). Catalyzed reactions have a lower activation energy (rate-limiting free energy of activation) than the corresponding uncatalyzed reaction, resulting in a higher reaction rate at the same temperature. Catalysts may affect the reaction environment favorably, or bind to the reagents to polarize bonds, or form specific intermediates that are not typically produced by a uncatalyzed reaction, or cause dissociation of reagents to reactive forms.

The invention is not intended to be limited in any manner by the above exemplary listing of substituents.

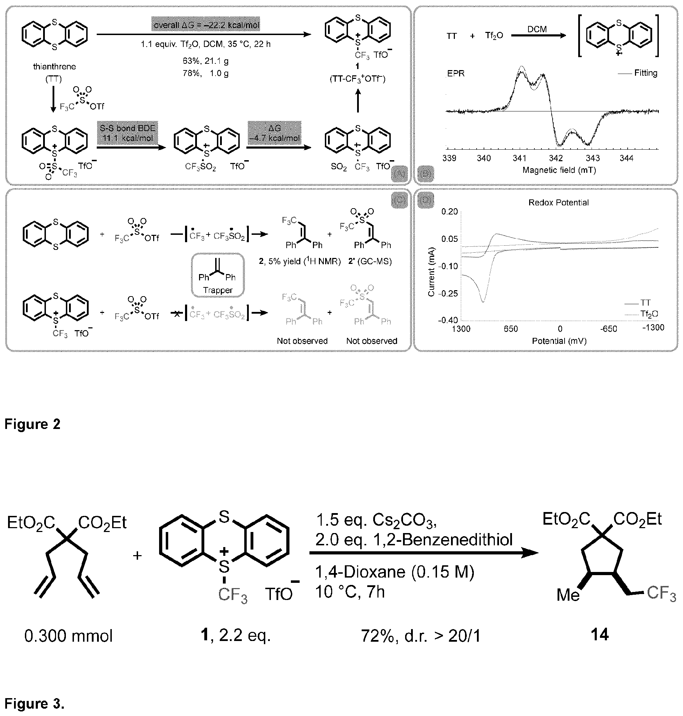

Surprisingly, the inventors found that simple mixing of thianthrene with triflic anhydride in DCM under ambient conditions yields trifluoromethylated thianthrene (1, TT-CF₃⁺OTf⁻). The reaction does not require an inert atmosphere, can be scaled up, and the product can be purified by washing with diethyl ether to deliver an easy to handle, free-flowing off-white powder that can be stored under ambient conditions in the absence of light without significant decomposition for at least a year (as illustrated in Figure 2A).

An analogous reaction of triflic anhydride with dibenzothiophene cannot be used for a practical synthesis of the *Umemoto's* reagent. The inventors rationalize the unanticipated reaction for the formation of TT-CF₃⁺OTf⁻ from triflic anhydride by a mechanism that can proceed with a low barrier due to the stability of the thianthrene radical cation (as illustrated in Figure 2A): Nucleophilic attack of thianthrene to triflic anhydride results in S-S bond formation, which can cleave homolytically to release two radicals due to its low bond dissociation energy, computed at 11.1 kcal/mol. Sulfur dioxide expulsion results in formation of the trifluoromethyl radical that can recombine with the persistent thianthrenium radical cation to form TT-CF₃⁺OTf⁻. Control experiments support the presence of the thianthrenium radical cation (as illustrated in Figure 2B) and the presence of the trifluoromethylsulfinyl radical and trifluoromethyl radical before the reagent is formed (as illustrated in Figure 2C). Cyclic voltammetry measurements for thianthrene (TT, *E*ₒₓ = 0.86 V *vs* Cp₂Fe) and triflic anhydride indicate that single electron oxidation of thianthrene by triflic anhydride is unlikely (as illustrated in Figure 2D).

The invention is further illustrated by the attached Figures and Examples. In the Figures, the respective Figure shows:
- Fig. 1:: Common electrophilic CF₃ reagents, trifluoromethyl thianthrenium triflate (1, TT-CF₃⁺OTf⁻), and typical applications of TT-CF₃⁺OTf⁻;
- Fig. 2:: Synthesis of TT-CF₃⁺OTf⁻, and reactivity studies;
- Fig. 3:: Radical clock control experiment; and
- Fig. 4:: (A) Cross coupling reaction,
(B) electrophilic trifluoromethylation,
(C) radical trifluoromethylation,
(D) radical hydrotrifluoromethylation of olefins, and
(E) nucleophilic trifluoromethylation with TT-CF₃⁺OTf⁻.

In more detail, the Figures illustrate as follows:
Figure 1. Common electrophilic CF₃ reagents, trifluoromethyl thianthrenium triflate (1, TT-CF₃⁺OTf⁻), and typical applications of TT-CF₃⁺OTf⁻.

Figure 2. Synthesis of TT-CF₃⁺OTf⁻, and reactivity studies.
(A) Synthesis of TT-CF₃⁺OTf⁻ including a proposed mechanism for its formation. Δ*H* and Δ*G* data were derived from DFT studies. The bond dissociation energy (BDE) of S-S bond was represented by Δ*H*. (B) electron paramagnetic resonance spectrum under the reaction conditions (magnetic flux density), (C) CF₃ radical trapping experiments, and (D) cyclic voltammogram of thianthrene and triflic anhydride in CH₃CN *(E* [mV] *vs* Cp₂Fe).

Figure 3. Radical clock control experiment
14 was isolated and characterized as analytically pure samples. The d.r. value was determined by ¹H NMR.

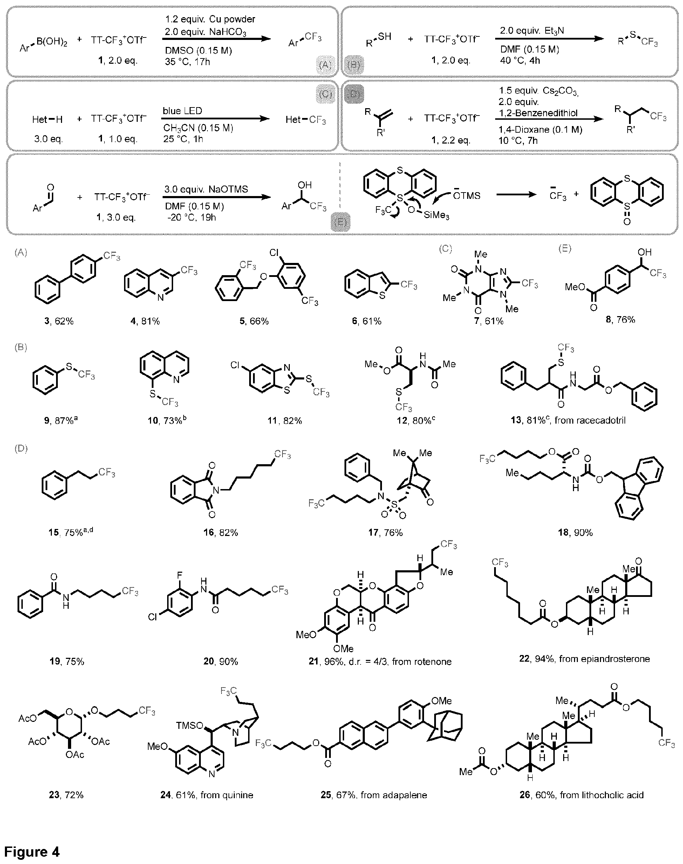

Figure 4. (A) Cross coupling reaction, (B) electrophilic trifluoromethylation, (C) radical trifluoromethylation, (D) radical hydrotrifluoromethylation of olefins, and (E) nucleophilic trifluoromethylation with TT-CF₃⁺OTf⁻.

The reaction conditions are marked in the reaction equation. All non-volatile trifluorometylation products were isolated and characterized as analytically pure samples. ^{a}Yields of volatile trifluorometylation products are reported based on ¹⁹F NMR integration of reaction mixtures with internal standard Ph-CF₃. ^{b}3.0 equiv. Et₃N were used, on account of starting hydrochloride material. ^{c}Aliphatic thiol (0.30 mmol, 1.0 equiv.), TT-CF₃⁺OTf⁻ (1.2 equiv.), 1,1,3,3-tetramethyl-guanidine (TMG, 1.2 equiv.), CH₃CN (2.0 mL c = 0.15 M), r.t., 6 h. ^{d}Styrene (1.00 mmol, 1.0 equiv.), TT-CF₃⁺OTf⁻ (1.5 equiv.), Cs₂CO₃ (2.0 equiv.), 1,2-benzenedithiol (2.0 equiv.), *i*-butanol (1.0 mL c = 0.10 M), 0 °C, 30 min.

### Experimental Part

All reactions were carried out under ambient atmosphere unless otherwise stated. High-resolution mass spectra were obtained using *Q Exactive Plus* from *Thermo.* Concentration under reduced pressure was performed by rotary evaporation at 23-40 °C at an appropriate pressure. Purified compounds were further dried under vacuum (10⁻⁶-10⁻³ bar). Yields refer to purified and spectroscopically pure compounds or mixtures of constitutional isomers. All air- and moisture-sensitive manipulations were performed using standard Schlenk- and glove-box techniques under an atmosphere of argon or dinitrogen.

### Solvents

Dichloromethane, anhydrous DMSO, and anhydrous DMF were purchased from Fisher Scientific GmbH. Anhydrous 1,4-dioxane and anhydrous acetonitrile were obtained from Phoenix Solvent Drying Systems. *i*-Butanol was degassed by three freeze-pump-thaw cycles. All deuterated solvents were purchased from Eur*iso*-Top.

### Chromatography

Thin layer chromatography (TLC) was performed using EMD TLC silica gel 60 F₂₅₄ plates pre-coated with 250 µm thickness silica gel 60 F₂₅₄ and visualized by fluorescence quenching under UV light, KMnO₄ stain or vanillin-H₂SO₄ stain. Flash column chromatography was performed using silica gel (40-63 µm particle size) purchased from Geduran. Preparatory high-performance liquid chromatographic separation was executed on a Shimadzu Prominence Preparative HPLC system with an YMC Pack Pro C18 HPLC column.

### Spectroscopy and instruments

NMR spectra were recorded on a Bruker *Ascend^{™}* 500 spectrometer operating at 500 MHz, 471 MHz, and 126 MHz, for ¹H, ¹⁹F, and ¹³C acquisitions, respectively, or on a Bruker *Ascend^{™}* 300 spectrometer operating at 300 MHz, 282 MHz, and 75 MHz, for ¹H, ¹⁹F, and ¹³C acquisitions, respectively. Chemical shifts are reported in ppm with the solvent residual peak as the internal standard.¹ For ¹H NMR: CDCl₃, δ 7.26; CD₃OD, δ 3.31; (CD₃)₂SO, δ 2.50; CD₃CN, δ 1.94, CD₂Cl₂, δ 5.32. For ¹³C NMR: CDCl₃, δ 77.16; CD₃OD, δ 49.00; (CD₃)₂SO, δ 39.52; CD₃CN, δ 1.32, CD₂Cl₂, δ 53.84. ¹⁹F NMR spectra were referenced using a unified chemical shift scale based on the ¹H NMR resonance of tetramethylsilane (1% (v/v) solution in the respective solvent).² Data is reported as follows: s = singlet, d = doublet, t = triplet, q = quartet, m = multiplet, br = broad; coupling constants in Hz; integration. For cyclic voltammetry a *MF-2013* electrode (99.95% Pt, 1.6 mm diameter) from *Bioanalytic systems Inc.* was used as working electrode.

### Starting materials

All substrates and materials were used as received from commercial suppliers, unless otherwise stated. For convenient operation, Cs₂CO₃ was stored in a N₂-filled glovebox after being dried at 150 °C for 36 h under high-vacuum. Substrates **S22,**³ **S23**,⁴ **S24,**⁵ and **S26**³ were prepared according to published procedures.

### X-rav crystallographic Analysis

A crystal was mounted on a nylon loop using perfluoropolyether, and transferred to a Bruker AXS Enraf-Nonius KappaCCD diffractometer (either MoKa radiation, λ=0.71073 Å or CuKa radiation, λ=1.54178 Å) equipped with an Oxford Cryosystems nitrogen flow apparatus. The sample was held at 100(2) K during the experiment. The structures were solved by the direct methods procedure and refined by invariom tool.⁶ Non-hydrogen atoms were refined anisotropically, and hydrogen atoms were allowed to ride on the respective atoms.

### S-(Trifluoromethyl)thianthrenium salts synthesis

### S-(Trifluoromethyl)thianthrenium triflate (1, TTCF₃⁺OTf⁻)

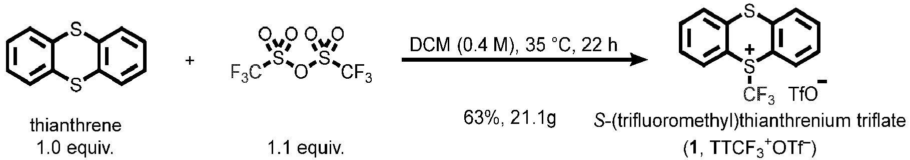

Under an ambient atmosphere, a 500 mL two-neck round-bottom flask equipped with a teflon-coated magnetic stirring bar, was charged with thianthrene (17.3 g, 80.0 mmol, 1.00 equiv.) and dichloromethane (200 mL, c = 0.4 M). Subsequently, triflic anhydride (24.8 g, 14.8 mL, 88.0 mmol, 1.10 equiv.) was added in one portion at room temperature. Upon addition of triflic anhydride, the reaction mixture rapidly turned light purple and gradually deepened, accompanied by formation of suspended particles. The reaction mixture was stirred at 35°C for 22 h. Subsequently, a saturated aqueous NaHCO₃ solution (ca. 100 mL) was added carefully. At this point, the purple color faded away, and the suspension turned light brown. The suspension was poured into a 500 mL separatory funnel, and the aqueous layer was discarded. The organic layer (light brown solution) was concentrated to dryness under reduced pressure, resulting in the formation of light brown residue. Diethyl ether (100 mL) was added to the residue and the suspension was stirred vigorously at room temperature for 30 min. The mixture was allowed to stand for 5 min, subsequently, the solvent was decanted carefully. In order to obtain an analytically pure compound, the decanting process was repeated four times with diethyl ether. The resulting yellow slurry was concentrated to dryness under reduced pressure, accumulating to a total of 21.1 g (63%) of S-(trifluoromethyl)thianthrenium triflate (1, TTCF₃⁺OTf⁻) as pale yellow solid. In most cases, S-(trifluoromethyl)thianthrenium triflate (1, TTCF₃⁺OTf⁻) can be used directly in subsequent transformations without chromatographic purification or recrystallization.
S-(Trifluoromethyl)thianthrenium triflate (1, TTCF₃⁺OTf⁻) can be purified by recrystallization using a mixture of DCM and n-pentane (approximately 1:1 (v:v)) or by chromatography on silica gel eluting with DCM:MeOH (30:1 (v:v)) to afford a colorless solid.
**R*_{f}***(DCM:MeOH, 10:1 (v:v)) = 0.35 (UV).
**Melting point:** 142-143 °C. Melting process is accompanied by decomposition possibly, see *Simultaneous thermal analysis* section for discussion.

### S-(Trifluoromethyl)thianthrenium tetrafluoroborate (1', TTCF₃⁻BF₄⁻)

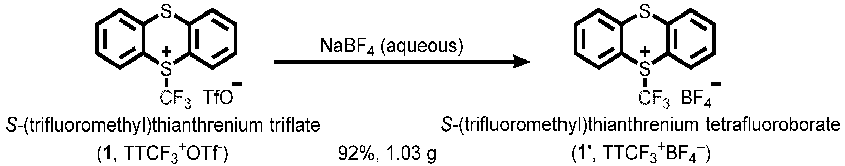

Under an ambient atmosphere, the S-(trifluoromethyl)thianthrenium triflate (1, TTCF₃⁺OTf⁻, 1.30 g, 3.00 mmol) was dissolved in dichloromethane (20 mL, c = 0.15 M). The dichloromethane solution was washed with aqueous NaBF₄ solution (c = 10% (w/v), 4 x 20 mL), dried over anhydrous MgSO₄, filtered, and the solvent was removed under reduced pressure. The title product (**1'** TTCF₃⁺BF₄⁻) was obtained as yellow solid (1.03 g, 92%). **R*_{f}***(DCM:MeOH, 10:1 (v:v)) = 0.35 (UV).

### Trifluoromethylation of aryl boronic acid

### 4-(Trifluoromethyl)-1,1'-biphenyl (3)

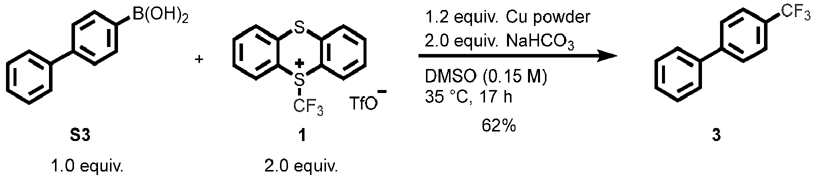

Under an ambient atmosphere, a 4 mL vial equipped with a teflon-coated magnetic stirring bar, was charged with 4-biphenylboronic acid (**S3**, 59.4 mg, 0.300 mmol, 1.00 equiv.), trifluoromethyl thianthrenium triflate (**1**, 261 mg, 0.600 mmol, 2.00 equiv.), Cu powder (22.9 mg, 0.360 mmol, 1.20 equiv.), and NaHCO₃ (50.4 mg, 0.600 mmol, 2.00 equiv.). The vial was transferred into a N₂-filled glovebox. Subsequently, dry DMSO (2 mL, c = 0.15 M) was added into the vial. The vial was capped, then it was transferred out of the glovebox. The reaction mixture was stirred at 35 °C for 17 h. The reaction mixture was added into 20 mL EtOAc in a separatory funnel, then the organic phase was washed with water (3 x 10 mL), dried over Na₂SO₄, and concentrated to roughly 3 mL under reduced pressure. Silica gel (approximately 500 mg) was added, and the mixture was evaporated to dryness. The residue was purified by column chromatography on silica gel eluting with pentane to afford 41.0 mg of the title compound (3) as colorless solid (62% yield).
**R*_{f}*** (pentane) = 0.55 (UV).

### 3-(Trifluoromethyl)quinoline (4)

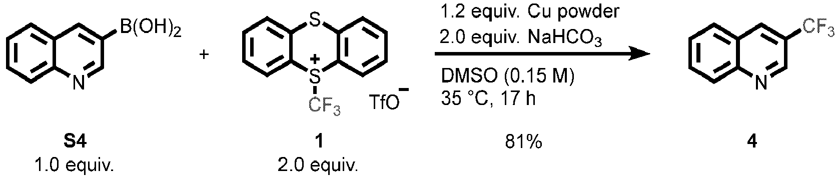

Under an ambient atmosphere, a 4 mL vial equipped with a teflon-coated magnetic stirring bar, was charged with quinolin-3-ylboronic acid (**S4**, 51.9 mg, 0.300 mmol, 1.00 equiv.), trifluoromethyl thianthrenium triflate (**1**, 261 mg, 0.600 mmol, 2.00 equiv.), Cu powder (22.9 mg, 0.360 mmol, 1.20 equiv.), and NaHCO₃ (50.4 mg, 0.600 mmol, 2.00 equiv.). The vial was transferred into a N₂-filled glovebox. Subsequently, dry DMSO (2 mL, c = 0.15 M) was added into the vial. The vial was capped, then it was transferred out of the glovebox.

The reaction mixture was stirred at 35 °C for 17 h. The reaction mixture was added into 20 mL EtOAc in a separatory funnel, then the organic phase was washed with water (3 x 10 mL), dried over Na₂SO₄, then filtered. The solvent was removed under reduced pressure. The residue was purified by column chromatography on silica gel eluting with a solvent mixture of EtOAc:pentane (1:25 (v:v)) to afford 47.9 mg of the title compound (**4**) as colorless solid (81% yield).
**R*_{f}*** (EtOAc:pentane, 1:4 (v:v)) = 0.21 (UV).
8.0, 6.8, 0.9 Hz, 1H).

### 1-Chloro-4-(trifluoromethyl)-2-((2-(trifluoromethyl)benzyl)oxy)benzene (5)

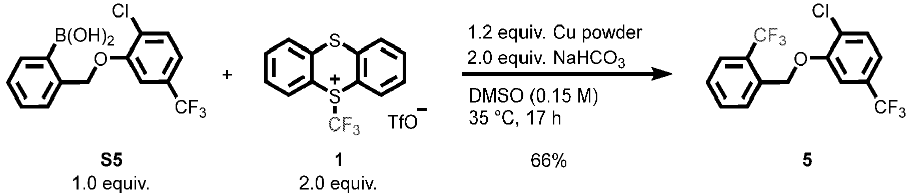

Under an ambient atmosphere, a 4 mL vial equipped with a teflon-coated magnetic stirring bar, was charged with (2-((2-chloro-5-(trifluoromethyl)phenoxy)methyl)phenyl)boronic acid (**S5**, 99.1 mg, 0.300 mmol, 1.00 equiv.), trifluoromethyl thianthrenium triflate (**1**, 261 mg, 0.600 mmol, 2.00 equiv.), Cu powder (22.9 mg, 0.360 mmol, 1.20 equiv.), and NaHCO₃ (50.4 mg, 0.600 mmol, 2.00 equiv.). The vial was transferred into a N₂-filled glovebox. Subsequently, dry DMSO (2 mL, c = 0.15 M) was added into the vial. The vial was capped, then it was and transferred out from the glovebox. The reaction mixture was stirred at 35 °C for 17 h. The reaction mixture was added into 20 mL EtOAc in a separatory funnel, then the organic phase was washed with water (3 x 10 mL), dried over Na₂SO₄, and concentrated to roughly 3 mL under reduced pressure. Silica gel (approximately 500 mg) was added, and the mixture was evaporated to dryness. The residue was purified by column chromatography on silica gel eluting with pentane to afford 70.1 mg of the title compound (**5**) as colorless solid (66% yield).
**R*_{f}*** (pentane) = 0.54 (UV).

### 2-(Trifluoromethyl)benzo[b]thiophene (6)

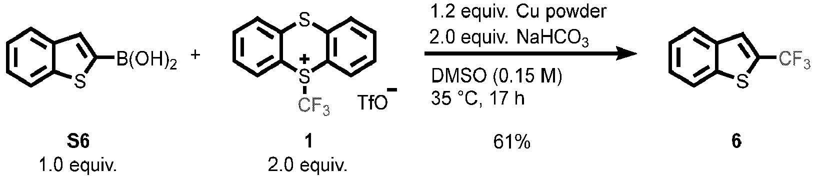

Under an ambient atmosphere, a 4 mL vial equipped with a teflon-coated magnetic stirring bar, was charged with benzo[b]thiophen-2-ylboronic acid (**S6**, 53.4 mg, 0.300 mmol, 1.00 equiv.), trifluoromethyl thianthrenium triflate (**1**, 261 mg, 0.600 mmol, 2.00 equiv.), Cu powder (22.9 mg, 0.360 mmol, 1.20 equiv.), and NaHCO₃ (50.4 mg, 0.600 mmol, 2.00 equiv.). The vial was transferred into a N₂-filled glovebox. Subsequently, dry DMSO (2 mL, c = 0.15 M) was added into the vial. The vial was capped, then it was transferred out of the glovebox. The reaction mixture was stirred at 35 °C for 17 h. The reaction mixture was added into 20 mL EtOAc in a separatory funnel, then the organic phase was washed with water (3 x 10 mL), dried over Na₂SO₄, and concentrated to roughly 3 mL under reduced pressure. Silica gel (approximately 500 mg) was added, and the mixture was evaporated to dryness. The residue was purified by column chromatography on silica gel eluting with pentane to afford 37.0 mg of the title compound (6) as colorless solid (61 % yield).
**R*_{f}*** (pentane) = 0.62 (UV).

### Trifluoromethylation of caffeine

### Caffeine derivative 7

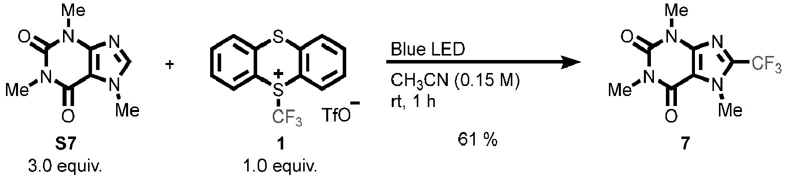

Under an ambient atmosphere, a 4 mL vial containing a teflon-coated magnetic stirring bar, was charged with caffeine (**S7**, 0.900 mmol, 174 mg, 3.00 equiv.) and trifluoromethyl thianthrenium triflate (1, 0.300 mmol, 130 mg, 1.00 equiv.). The vial was transferred into a N₂-filled glovebox. The vial was capped, then it was transferred out of the glovebox. After adding dry CH₃CN (2 mL, c = 0.15 M), the reaction mixture was stirred under blue LED (34 W) at room temperature for 1 h. The solvent was removed under reduced pressure. The residue was purified by column chromatography on silica gel eluting with a solvent mixture of EtOAc:pentane (1:15 gradient to 1:5 (v:v)) to afford 48.0 mg of the title compound (**7**) as colorless solid (61% yield).
**R*_{f}*** (EtOAc:pentane, 1:3 (v:v)) = 0.32 (UV).

### Trifluoromethylation of aryl aldehyde

### Methyl 4-(2,2,2-trifluoro-1-hydroxyethyl)benzoate (8)

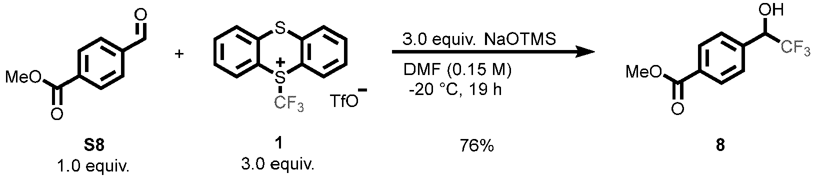

Under an ambient atmosphere, a 4 mL vial containing a teflon-coated magnetic stirring bar, was charged with methyl 4-formylbenzoate (**S8**, 49.2 mg, 0.300 mmol, 1.00 equiv.), trifluoromethyl thianthrenium triflate (**1**, 391 mg, 0.900 mmol, 3.00 equiv.), and NaOTMS (101 mg, 0.900 mmol, 3.00 equiv.). The vial was transferred into a N₂-filled glovebox. Subsequently the vial was capped, then it was transferred out of the glovebox. After adding dry DMF (2 mL, c = 0.15 M), the reaction mixture was stirred at -20 °C for 19 h. EtOAc (20 mL) was added and the resulting mixture was transferred to a separating funnel. The organic phase was washed with water (3 x 10 mL), dried over Na₂SO₄, and concentrated to roughly 3 mL under reduced pressure. Silica gel (approximately 500 mg) was added, and the mixture was evaporated to dryness. The residue was purified by column chromatography on silica gel eluting with EtOAc:pentane (1:70 gradient to 1:10 (v:v)) to afford 53.4 mg of the title compound (**8**) as colorless solid (76% yield).
**R*_{f}*** (EtOAc:pentane, 1:4 (v:v)) = 0.26 (UV).

### Trifluoromethylation of thiols

### Phenyl(trifluoromethyl)sulfane (9)

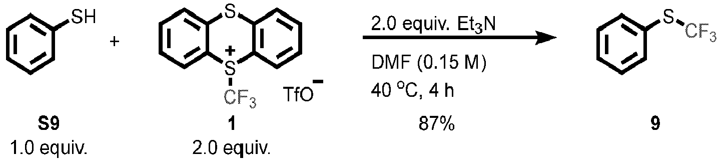

Under an ambient atmosphere, a 4 mL vial containing a teflon-coated magnetic stirring bar, was charged with trifluoromethyl thianthrenium triflate (1, 261 mg, 0.600 mmol, 2.00 equiv.). The vial was transferred into a N₂-filled glovebox. Subsequently, dry DMF (2 mL, c = 0.15 M) was added into the vial. The vial was sealed with a teflon-lined screw cap and removed from the glovebox. After adding thiophenol (**S9**, 33.1 mg, 30.80 µL, 0.300 mmol, 1.00 equiv.) and Et₃N (91.1 mg, 125 µL, 0.900 mmol, 3.00 equiv.), the reaction mixture was stirred at 40 °C for 4 h. Subsequently, trifluorotoluene (43.8 mg, 40.0 µL, 0.300 mmol, 1.00 equiv.) was added as an internal standard. The reaction mixture was diluted with CDCl₃, and the yield was determined by ¹⁹F NMR spectroscopy by integration of the peak at δ -62.91 ppm of the internal standard and the peak at δ -43.28 ppm of the product (9, 87% yield). The identity of the product was further confirmed by GC-MS analysis.

### 8-((Trifluoromethyl)thio)quinolone (10)

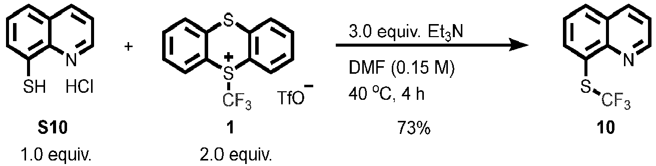

Under an ambient atmosphere, a 4 mL vial containing a teflon-coated magnetic stirring bar, was charged with thiooxine hydrochloride (**S10**, 59.3 mg, 0.300 mmol, 1.00 equiv.) and trifluoromethyl thianthrenium triflate (**1**, 261 mg, 0.600 mmol, 2.00 equiv.). The vial was transferred into a N₂-filled glovebox. Subsequently, dry DMF (2 mL, c = 0.15 M) and Et₃N (91.1 mg, 125 µL, 0.900 mmol, 3.00 equiv.) was added into the vial. The vial was sealed with a teflon-lined screw cap and removed from the glovebox. The reaction mixture was stirred at 40 °C for 4 h. EtOAc (20 mL) was added and the resulting mixture was transferred to a separating funnel. The organic phase was washed with water (3 x 10 mL). The organic phase was dried over Na₂SO₄, and the solvent was removed under reduced pressure. The residue was purified by column chromatography on silica gel eluting with a solvent mixture of EtOAc:pentane (1:20 (v:v)) to afford 49.9 mg of the title compound (**10**) as colorless liquid (73% yield).
**R*_{f}*** (EtOAc:pentane, 1:5 (v:v)) = 0.48 (UV).

### 5-Chloro-2-((trifluoromethyl)thio)benzo[d]thiazole (11)

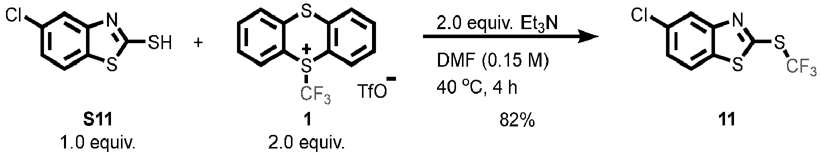

Under an ambient atmosphere, a 4 mL vial containing a teflon-coated magnetic stirring bar, was charged with 5-chloro-2-benzothiazolethiol (**S11**, 60.5 mg, 0.300 mmol, 1.00 equiv.) and trifluoromethyl thianthrenium triflate (**1**, 261 mg, 0.600 mmol, 2.00 equiv.). The vial was transferred into a N₂-filled glovebox. Subsequently, dry DMF (2 mL, c = 0.15 M) and Et₃N (60.7 mg, 83.6 µL, 0.600 mmol, 2.00 equiv.) were added into the vial. The vial was sealed with a teflon-lined screw cap and removed from the glovebox. The reaction mixture was stirred at 40 °C for 4 h. EtOAc (20 mL) was added and the resulting mixture was transferred to a separating funnel. The organic phase was washed with water (3 x 10 mL), dried over Na₂SO₄, and concentrated to roughly 3 mL under reduced pressure. Silica gel (approximately 500 mg) was added, and the mixture was evaporated to dryness. The residue was purified by column chromatography on silica gel eluting with pentane to afford 66.1 mg of the title compound (**11**) as colorless solid (82% yield).
**R*_{f}*** (pentane) = 0.18 (UV).

### Methyl N-acetyl-S-(trifluoromethyl)-L-cysteinate (12)

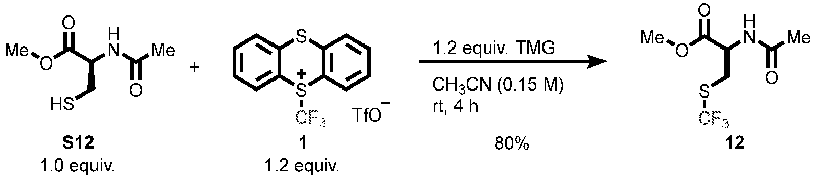

Under an ambient atmosphere, a 4 mL vial containing a teflon-coated magnetic stirring bar, was charged with methyl acetyl-L-cysteinate (**S12**, 53.2 mg, 0.300 mmol, 1.00 equiv.) and trifluoromethyl thianthrenium triflate (**1**, 156 mg, 0.360 mmol, 1.20 equiv.). The vial was transferred into a N₂-filled glovebox. Subsequently, dry CH₃CN (2 mL, c = 0.15 M) and 1,1,3,3-tetramethylguanidin (TMG, 41.5 mg, 45.2 µL, 0.360 mmol, 1.20 equiv.) were added into the vial. The vial was sealed with a teflon-lined screw cap and removed from the glovebox. The reaction mixture was stirred at room temperature for 4 h. The solvent was removed under reduced pressure. The residue was purified by column chromatography on silica gel eluting with a solvent mixture of EtOAc:pentane (1:5 gradient to 1:2 (v:v)) to afford 59.4 mg of the title compound (**12**) as colorless solid (80% yield).
**R*_{f}*** (EtOAc:pentane, 1:1 (v:v)) = 0.24 (KMnO₄) .

### Racecadotril derivative 13

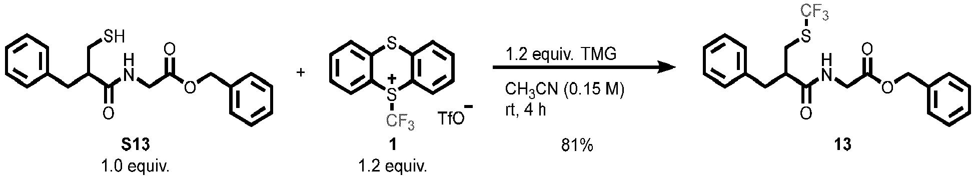

Under an ambient atmosphere, a 4 mL vial containing a teflon-coated magnetic stirring bar, was charged with racecadotril derived thiol (**S13**, 103 mg, 0.300 mmol, 1.00 equiv.) and trifluoromethyl thianthrenium triflate (**1**, 156 mg, 0.360 mmol, 1.20 equiv.). The vial was transferred into a N₂-filled glovebox. Subsequently, dry CH₃CN (2 mL, c = 0.15 M) and 1,1,3,3-tetramethylguanidin (TMG, 41.5 mg, 45.2 µL, 0.360 mmol, 1.20 equiv.) were added into the vial. The vial was sealed with a teflon-lined screw cap and removed from the glovebox. The reaction mixture was stirred at room temperature for 4 h. The solvent was removed under reduced pressure. The residue was purified by column chromatography on silica gel eluting with a solvent mixture of EtOAc:pentane (1:15 gradient to 1:8 (v:v)) to afford 80.8 mg of the title compound (**13**) as colorless solid (81% yield).
**R*_{f}*** (EtOAc:pentane, 1:2 (v:v)) = 0.59 (UV).

### Hydrotrifluoromethylation of olefins

### Diethyl 3-methyl-4-(2,2,2-trifluoroethyl)cyclopentane-1,1-dicarboxylate (14)

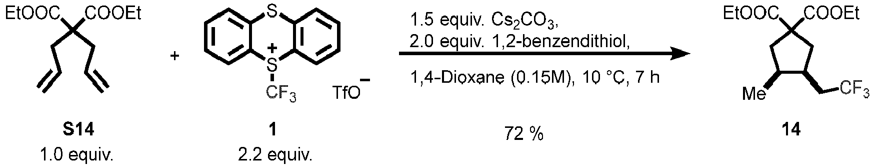

Under an ambient atmosphere, a 4 mL vial containing a teflon-coated magnetic stirring bar, was charged with trifluoromethyl thianthrenium triflate (**1**, 287 mg, 0.660 mmol, 2.20 equiv.). The vial was transferred into a N₂-filled glovebox. Subsequently, Cs₂CO₃ (147 mg, 0.450 mmol, 1.50 equiv.) was added into the vial. The vial was capped, then it was transferred out of the glovebox. After adding dry 1,4-dioxane (2 mL, c = 0.15 M), the reaction mixture was stirred at 10 °C for 1 min, followed by addition of radical clock reagent (**S14**, 72.1 mg, 0.300 mmol, 1.00 equiv.) and 1,2-benzenedithiol (85.3 mg, 69.1 µL, 0.600 mmol, 3.00 equiv.) with a Hamilton syringe. The reaction mixture was stirred at 10°C for 7 h. Subsequently, the reaction mixture was filtered (0.2 µm PTFE), and the residue was washed with DCM (0.5 mL). Silica gel (approximately 600 mg) was added into the combined transparent organic phase. Then the mixture was concentrated to dryness under reduced pressure. The residue was purified by column chromatography on silica gel eluting with a solvent mixture of EtOAc:pentane (pure pentane gradient to 1:50 (v:v)) to afford 66.6 mg of the title compound (**14**) as yellow liquid (72% yield). The d.r. (>20:1) value was determined by ¹H NMR. The configuration of the product was determined by comparing with the literature.⁷
**R*_{f}*** (EtOAc:pentane, 1:9 (v:v)) = 0.42 (KMnO₄, very weak).

### (3,3,3-Trifluoropropyl)benzene (15)

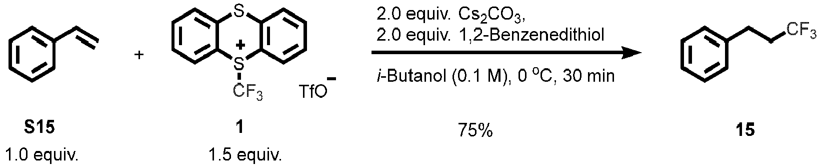

Under an ambient atmosphere, a 20 mL vial containing a teflon-coated magnetic stirring bar, was charged with trifluoromethyl thianthrenium triflate (**1**, 651 mg, 1.50 mmol, 1.50 equiv.). The vial was transferred into a N₂-filled glovebox. Subsequently, Cs₂CO₃ (651 mg, 2.00 mmol, 2.00 equiv.) was added into the vial. The vial was capped, then it was transferred out of the glovebox. After adding dry *i*-butanol (10 mL, c = 0.1 M), the reaction mixture was stirred at 0 °C for 2 min, followed by addition of styrene (**S15**, 104 mg, 115 µL, 1.00 mmol, 1.00 equiv.) and 1,2-benzenedithiol (56.9 mg, 46.0 µL, 0.400 mmol, 2.00 equiv.). The reaction mixture was stirred at 0°C for 30 min. Subsequently, trifluorotoluene (292 mg, 246 µL, 1.00 mmol, 1.00 equiv.) was added as an internal standard. The reaction mixture was diluted with CDCl₃, and the yield was determined by ¹⁹F NMR spectroscopy by integration of the peak at δ -62.91 ppm of the internal standard and the peak at δ -66.88 (t, *J* = 10.2 Hz) ppm of the product (**15**, 75% yield). The identity of the product was further confirmed by GC-MS analysis.

### 2-(6,6,6-Trifluorohexyl)isoindoline-1,3-dione (16)

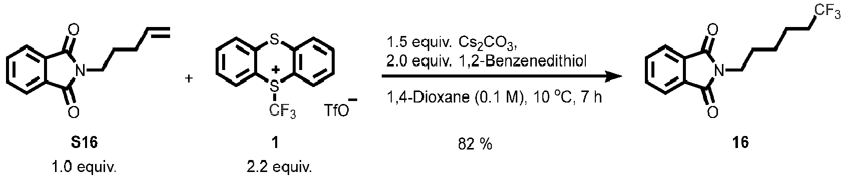

Under an ambient atmosphere, a 4 mL vial containing a teflon-coated magnetic stirring bar, was charged with phthalimide-derived olefin (**S16,** 43.0 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10°C for 1 min, followed by addition of 1,2-benzenedithiol (56.9 mg, 46.0 µL, 0.400 mmol, 2.00 equiv.). The reaction mixture was stirred at 10 °C for 7 h. Subsequently, the reaction mixture was filtered (0.2 µm, PTFE), and the residue was washed with DCM (0.5 mL). Silica gel (approximately 500 mg) was added into the combined transparent organic phase. Then this mixture was concentrated to dryness under reduced pressure. The residue was purified by column chromatography on silica gel eluting with a solvent mixture of EtOAc:pentane (1:30 (v:v)) to afford 47.0 mg of the title compound (**16**) as yellow liquid (82% yield).
**R*_{f}*** (EtOAc:pentane, 1:8 (v:v)) = 0.34 (UV).

### CF₃-(1S)-10-camphorsulfonamide (17)

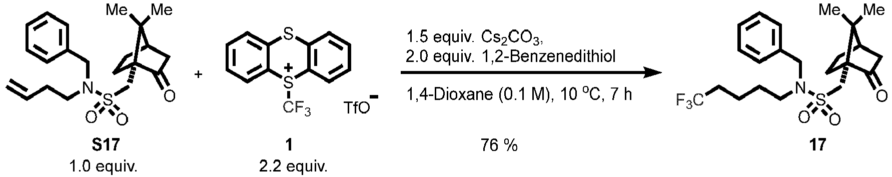

Under an ambient atmosphere, a 4 mL vial containing a teflon-coated magnetic stirring bar, was charged with trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of (1*S*)-10-camphorsulfonamide derived olefin (**S17**, 75.1 mg, 0.200 mmol, 1.00 equiv.) and 1,2-benzenedithiol (56.9 mg, 46.0 µL, 0.400 mmol, 2.00 equiv.) with a Hamilton syringe. The reaction mixture was stirred at 10 °C for 7 h. Subsequently, the reaction mixture was filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:20 (v:v)) to afford 67.7 mg of the title compound (**17**) as colorless liquid (76% yield).
**R*_{f}*** (EtOAc:pentane, 1:4 (v:v)) = 0.34 (UV).

### CF₃-Fmoc-L-Nle-OH derivative 18

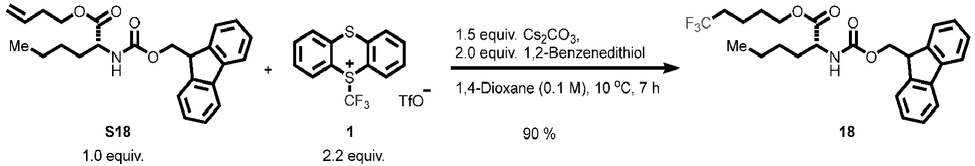

Under an ambient atmosphere, a 4 mL vial containing a teflon-coated magnetic stirring bar, was charged with Fmoc-L-Nle-OH-ester derived olefin (**S18**, 78.3 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h. Subsequently, the reaction mixture was filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:30 (v:v)) to afford 67.7 mg of the title compound (**18**) as slightly yellow solid (90% yield).
**R*_{f}*** (EtOAc:pentane, 1:6 (v:v)) = 0.24 (UV).

### N-(5,5,5-Trifluoropentyl)benzamide (19)

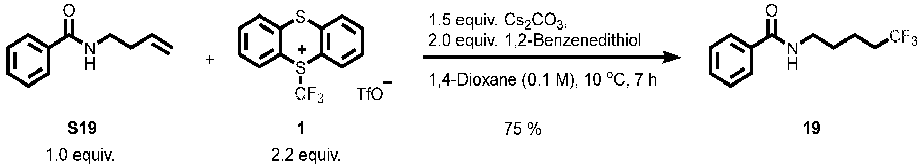

Under an ambient atmosphere, a 4 mL vial containing a teflon-coated magnetic stirring bar, was charged with benzamide derived olefin (**S19**, 35.0 mg, 0.200 mmol, 1.00 equiv.) and trifluoromethyl thianthrenium triflate (1, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10°C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h. Subsequently, the reaction mixture was filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:30 gradient to 1:5 (v:v)) to afford 36.7 mg of the title compound (**19**) as colorless solid (75% yield).
**R*_{f}*** (EtOAc:pentane, 1:2 (v:v)) = 0.29 (UV).

### N-(4-Chloro-2-fluorophenyl)-6,6,6-trifluorohexanamide (20)

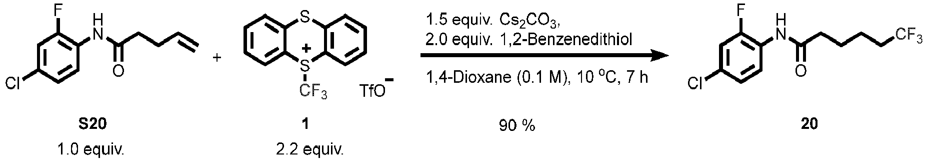

Under an ambient atmosphere, a 4 mL vial containing a teflon-coated magnetic stirring bar, was charged with *N*-(4-chloro-2-fluorophenyl)pent-4-enamide (**S20**, 35.0 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h. Subsequently, the reaction mixture was filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:30 gradient to 1:15 (v:v)) to afford 53.4 mg of the title compound (**20**) as colorless solid (90% yield).
**R*_{f}*** (EtOAc:pentane, 1:4 (v:v)) = 0.39 (UV).

### Rotenone derivative 21

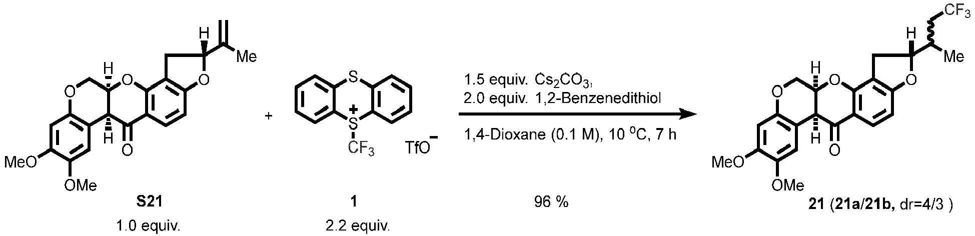

Under an ambient atmosphere, a 4 mL vial containing a teflon-coated magnetic stirring bar, was charged with rotenone (**S21**, 78.9 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:15 gradient to 1:5 (v:v)) to afford 89.4 mg of the title compound (**21**) as yellow solid (96% yield, a pair of diastereoisomers **21a** and **21b**), the d.r. value (4/3) was determined by ¹⁹F NMR. Further purification of **21** by preparative HPLC (YMC-Actus Pro C18 (30x150 mm: 5 µm), CH₃CN/H₂O = 48:52, flow rate = 42.5 mL/min, 23 °C) provided **21a** (retention time: 47.6 min) and **21b** (retention time: 49.9 min). **21a** or **21b** cannot be separated under this HPLC method completely. Pure **21a** and **21b** were confirmed by NMR and MS. Attempts have been made to determine the absolute configuration by NMR and growing single crystals, but without success.
**R*_{f}*** (EtOAc:pentane, 1:2 (v:v)) = 0.56 (UV).

### Epiandrosterone derivative 22

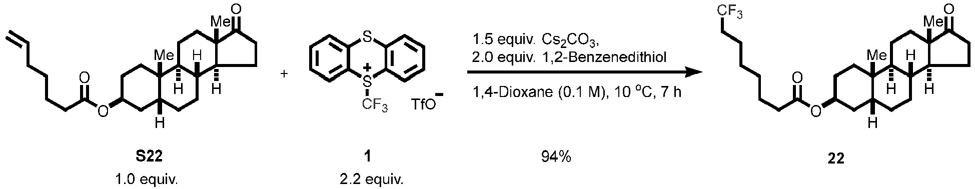

Under an ambient atmosphere, a 4 mL vial containing a teflon-coated magnetic stirring bar, was charged with trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of epiandrosterone esterderived olefin (**S22**, 80.1 mg, 0.200 mmol, 1.00 equiv.) and 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:40 gradient to 1:13 (v:v)) to afford 88.5 mg of the title compound (**22**) as colorless liquid (94% yield).
**R*_{f}*** (EtOAc:pentane, 1:7 (v:v)) = 0.39 (Vanillin-H₂SO₄).

### D-Glucose derivative 23

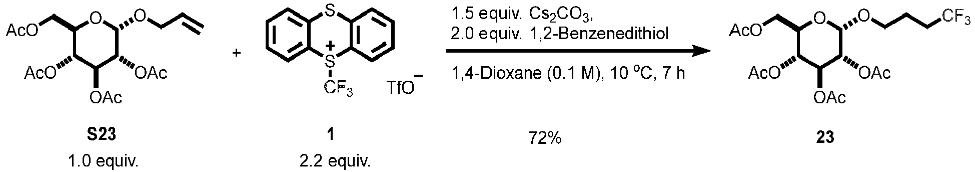

Under an ambient atmosphere, a 4 mL vial containing a teflon-coated magnetic stirring bar, was charged with D-glucose ester (**S23**, 77.7 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10°C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). The combined solvent was removed under reduced pressure. The residue was purified by column chromatography on silica gel eluting with a solvent mixture of EtOAc:pentane (1:15 gradient to 1:5 (v:v)) to afford 66.0 mg of the title compound (**23**) as colorless oil (72% yield). **R*_{f}*** (EtOAc:pentane, 1:2 (v:v)) = 0.35 (KMnO₄) .

### Quinine derivative 24

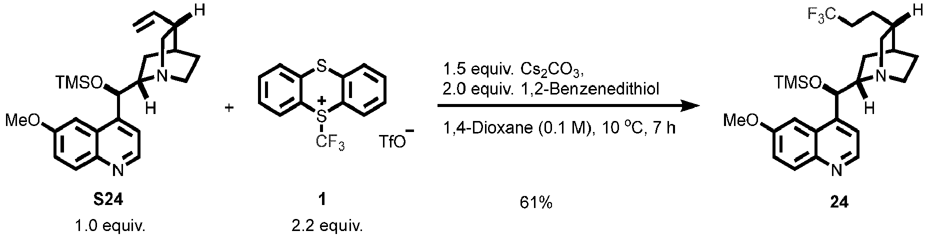

Under an ambient atmosphere, a 4 mL vial containing a teflon-coated magnetic stirring bar, was charged with TMS-quinine (**S24**, 67.7 mg, 0.200 mmol, 1.00 equiv.) and trifluoromethyl thianthrenium triflate (1, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). The combined solvent was removed under reduced pressure. The residue was purified by column chromatography on silica gel eluting with a solvent mixture of CH₃OH:CH₃CN (1:40 (v:v)) to afford 60.6 mg of the title compound (**24**) as colorless solid (61% yield).
**R*_{f}*** (CH₃OH:CH₃CN, 1:9 (v:v)) = 0.50 (UV).

### Adapalene derivative 25

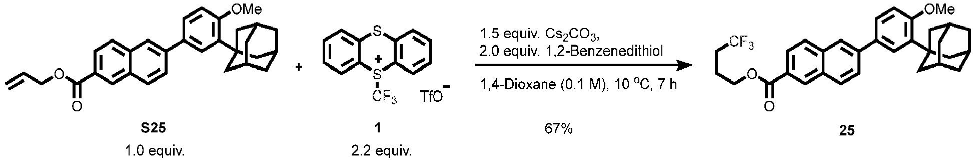

Under an ambient atmosphere, a 4 mL vial containing a teflon-coated magnetic stirring bar, was charged with adapalene allyl ester (**S25**, 90.5 mg, 0.200 mmol, 1.00 equiv.) and trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10°C for 1 min, followed by addition of 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). To the flask, add silica gel (approximately 500 mg), and concentrated to dryness under reduced pressure. The residue was purified by column chromatography on silica gel eluting with a solvent mixture of EtOAc:pentane (1:50 (v:v)) to afford 70.0 mg of the title compound (**25**) as colorless solid (67% yield).
**R*_{f}*** (EtOAc:pentane, 1:9 (v:v)) = 0.38 (UV).

### Lithocholic acid derivative 26

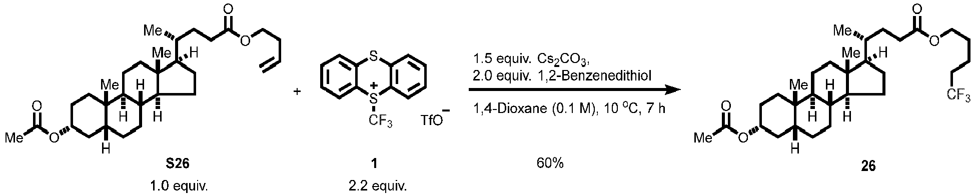

Under an ambient atmosphere, a 4 mL vial containing a teflon-coated magnetic stirring bar, was charged with trifluoromethyl thianthrenium triflate (**1**, 191 mg, 0.440 mmol, 2.20 equiv.). The vial was transferred into a N₂-filled glovebox. Subsequently, Cs₂CO₃ (97.7 mg, 0.300 mmol, 1.50 equiv.) was added into the vial. The vial was capped, then it was transferred out of the glovebox. After adding dry 1,4-dioxane (2 mL, c = 0.1 M), the reaction mixture was stirred at 10 °C for 1 min, followed by addition of lithocholic acid derived olefin (**S26**, 94.5 mg, 0.200 mmol, 1.00 equiv.) and 1,2-benzenedithiol (56.9 mg, 46.0 µL, 0.400 mmol, 2.00 equiv.) with a Hamilton syringe. The reaction mixture was stirred at 10 °C for 7 h, then filtered (0.2 µm, PTFE), and the residue was washed with DCM (0.5 mL). To the flask, add silica gel (approximately 500 mg), and concentrated to dryness under reduced pressure. The residue was purified by column chromatography on silica gel eluting with a solvent mixture of EtOAc:pentane (1:40 gradient to 1:20 (v:v)) to afford 67.5 mg of the title compound (**26**) as yellow oil (60% yield).
**R*_{f}*** (EtOAc:pentane, 1:0 (v:v)) = 0.42 (vanillin-H₂SO₄).

As illustrated above, the inventors have established that TT-CF₃⁺X⁻ , in particular TT-CF₃⁺OTf⁻, is a competent electrophilic trifluoromethylating reagent. For example, copper(0)-mediated cross coupling with arylboronic acids proceeds well (as illustrated in Figure 4A), as does electrophilic trifluoromethylation of sulfides (as illustrated in Figure 4B). As expected, TT-CF₃⁺OTf⁻ can also perform in radical trifluoromethylation as shown in Figure 4C. Unlike other electrophilic trifluoromethylation reagents, the inventors have shown that TT-CF₃⁺OTf⁻ is useful for nucleophilic trifluoromethylation (as illustrated in Figure 4E, left side) through umpolung with silanolate as nucleophile. The inventors explain this reactivity by formal expulsion of a trifluoromethyl anion with thianthrene oxide as side product upon addition of silanolate to the reagent to form a metastable sulfurane (as illustrated in Figure 4E, right side).

To evaluate TT-CF₃⁺OTf⁻ in a transformation that has not already been disclosed with other trifluoromethylating reagents, the inventors explored the hydrotrifluoromethylation of olefins in the absence of catalyst. Hydrotrifluoro-methylation with conventional trifluoromethylating reagents is known but only with catalysts, for example photoredox catalysts.²⁰ The inventors show here that hydrotrifluoromethylation of unactivated alpha olefins can be accomplished by simply adding olefin and TT-CF₃⁺OTf⁻ in the presence of 1,2-benzenedithiol as hydrogen atom donor (as illustrated in Figure 4D). The substrate scope of the hydrotrifluoromethylation and the functional group tolerance of the new reagent are large: esters, ethers, nitrogen heterocycles, and amides, as well as complex small molecules are all tolerated. Moreover, styrene derivatives, which often engage in unproductive polymerization, isomerization, and dimerization,^{20b, 21} are tolerated in the transformation. Based on the observed regioselectivity and radical clock cyclization product (as illustrated in Figure 3), the inventors propose that the transformation proceeds by radical trifluoromethylation of the olefin followed by hydrogen atom abstraction of the intermediate secondary carbon radical from the hydrogen donor.

In conclusion, the inventors have developed a new trifluoromethylating reagent, trifluoromethyl thianthrenium triflate (**1**, TT-CF₃⁺OTf⁻), which is easily accessible from commercial starting materials in a single step. The new reagent can engage in electrophilic, nucleophilic, and radical reactions, and promises to be of synthetic utility.

## Claims

1. A thianthrene derivative of the Formula (I): 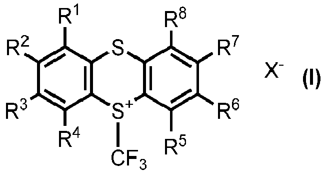 wherein R¹ to R⁸ may be the same or different and are each selected from hydrogen, halogen, or a C₁ to C₆ alkyl group, which may be substituted by at least one halogen, and wherein X⁻ is an anion, selected from F⁻, Cl⁻, triflate⁻, BF₄⁻, SbF₆⁻, PF₆⁻, ClO₄⁻, 0.5 SO₄²⁻, or NO₃⁻,

2. A thianthrene derivative of the Formula (I) as clamed in claim 1 wherein, in Formula (I), R¹ to R⁸ may be the same or different and are each selected from hydrogen, Cl or F and X⁻ is an anion as defined in claim 1, preferably triflate or BF₄⁻.

3. A thianthrene derivative of the Formula (I) as clamed in claim 1 wherein, in Formula (I), R¹ to R⁸ are each hydrogen, and X⁻ is an anion as defined in claim 1, preferably triflate or BF₄⁻.

4. Process for preparing a trifluoromethyl thianthrenium compound of the Formula (I), whereby a thianthrene derivative of the Formula (II) is reacted with a trifluoromethyl group transfer agent such as triflic acid anhydride in an organic solvent whereby a thianthrenium compound of the Formula (I) is obtained: 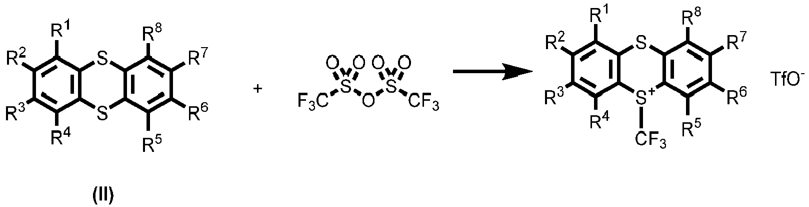 wherein R¹ to R⁸ may be the same or different and are each selected from hydrogen, halogen, or a C₁ to C₆ alkyl group, which may be substituted by at least one halogen.

5. Process for preparing the trifluoromethyl thianthrenium compound of the Formula (I) as claimed in claim 4, wherein the obtained trifluoromethyl thianthrenium triflate is treated with an anion exchange agent to obtain a trifluoromethyl)thianthrenium compound wherein the anion is selected from F⁻, Cl⁻, BF₄⁻, SbF₆⁻, PF₆⁻, ClO₄⁻, 0.5 SO₄²⁻, or NO₃⁻.

6. Use of a trifluoromethyl thianthrenium compound of the Formula (I) as a transfer agent for transferring a trifluoromethyl group to a hydrocarbon compound, in particular to an aliphatic hydrocarbon, an aromatic hydrocarbon or a heteroaromatic hydrocarbon.

7. Use of a trifluoromethyl thianthrenium compound of the Formula (I) as a transfer agent for transferring a trifluoromethyl group to a hydrocarbon compound according to claim 6, wherein said hydrocarbon compound has an at least one alkenyl moiety or C=O moiety and/or is substituted by at least one group selected from B(OR)₂;-SR or -OR wherein R is hydrogen or C₁ to C₃ alkyl.
